# EUROPEAN PATENT SPECIFICATION

(11) **EP 1 622 672 B1**
(45) Date of publication and mention of the grant of the patent: **29.06.2011**
(21) Application number: 04760977.1
(22) Date of filing: 11.05.2004
(51) Int. Cl.: A61M 25/10

(54) **NON-BUCKLING BALLOON CATHETER**
NICHT-KNICKENDER BALLONKATHETER
SONDE A BALLONNET SANS COURBURE

(30) Priority: 12.05.2003 US 436452; 02.03.2004 US 792031
(43) Date of publication of application: 08.02.2006
(73) Proprietor: Wilson-Cook Medical Inc., Winston-Salem, NC 27105-4191 (US)
(72) Inventor: KENNEDY, Kenneth, C., Clemmons, NC 27012 (US)
(74) Representative: Garratt, Peter Douglas
(86) International application number: PCT/US2004/014669
(87) International publication number: WO 2004/101059

(56) References cited:
- EP-A- 1 163 925
- WO-A-03/097152
- US-A- 5 681 344

## Description

### TECHNICAL FIELD

This invention relates to medical devices, and more particularly to balloon catheters that can be placed within a body lumen and inflated to perform various medical procedures. The invention is especially relevant to balloon catheters with balloons formed of non-elastomeric films or materials, wherein the film that forms the balloon is folded and unfolded during deflation and inflation, respectively, of the balloon.

### BACKGROUND OF THE INVENTION

Balloon catheters are used to perform various medical procedures wherein the balloon is positioned within a body lumen or canal and subsequently inflated. In some of these medical procedures, such as in an angioplasty procedure, the balloon is inflated so as to expand the interior volume of the body canal. In this type of procedure, the balloon is expanded to apply pressure to the interior surface of the body canal to thereby compress any tissue protruding into the canal and thereby enlarge the interior volume thereof. Once the tissue has been compressed, and the body canal widened, the balloon is deflated and removed.

In other types of medical procedures, such as photodynamic therapy (PDT), a balloon catheter is used to align and stabilize the catheter within the body lumen. For example, the balloon catheter may be inflated under low pressure within a body lumen such as the esophagus. A therapeutic fiber optic device is then inserted into the catheter in the vicinity of the balloon. The therapeutic fiber optic device is then used to emit light waves to treat the surrounding tissue. In this procedure, the balloon is used to both align the catheter in the center of the body lumen, and to prevent the catheter from moving during the PDT procedure. However, the tissue to be treated must not be unduly compressed by the expanded balloon. Thus, the balloon is expanded only enough to lightly contact the interior surface of the lumen and align the catheter.

As will be explained below, conventional balloon catheters have a number of shortcomings that make them inadequate for many of the above-described procedures, and in particular, for PDT procedures.

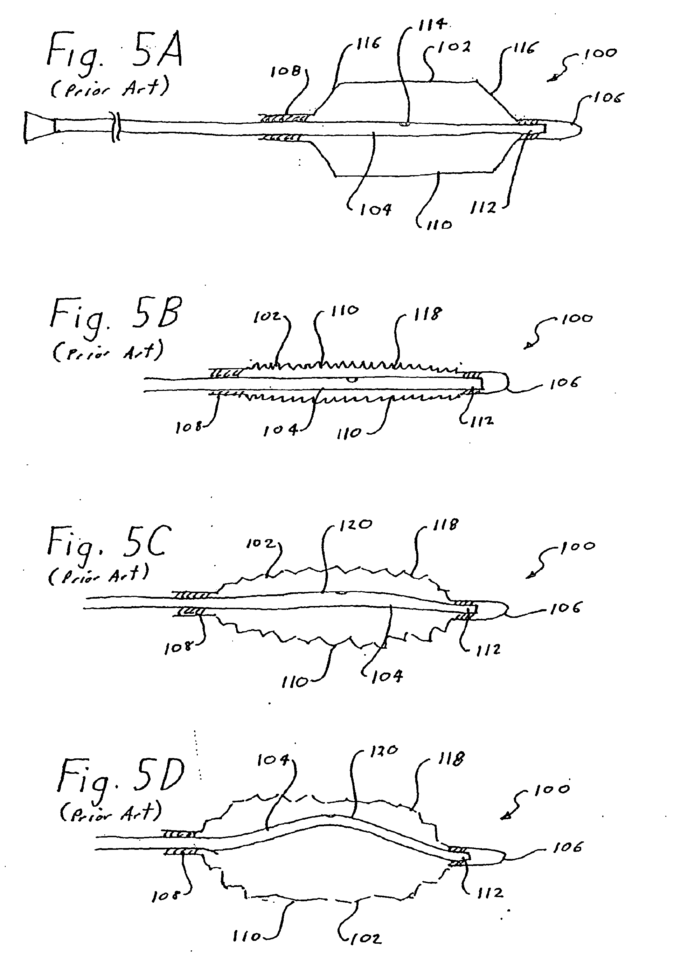

A typical balloon catheter 100 is shown in FIGS. 5A-5D. As best seen in FIG. 5A, a conventional balloon catheter 100 comprises a balloon 102 that is affixed to a catheter 104. The balloon 102 is typically manufactured from a non-elastomeric material (e.g., a semi-rigid or non-compliant material), and includes a distal neck or end 106, a proximal neck or end 108 and a central portion 110. The balloon 102 is affixed to the catheter 104 by inserting the distal end 112 of the catheter 104 into and through the proximal end 108 of the balloon 102. The balloon 102 is then slid over the catheter 104 until the distal end 112 of the catheter 104 is inserted into the distal end 106 of the balloon 102. The distal end 112 of the catheter 104 is then affixed to the distal end 106 of the balloon 102 by an adhesive, ultrasonic welding, or some other method. The proximal end 108 of the balloon 102 is similarly affixed to the outer wall of the catheter 104 so as to anchor and seal the proximal end of the balloon 102.

The catheter 104 includes an aperture 114 for the introduction of air or some other fluid into the interior volume of the balloon 102. Although not shown in the drawings, the proximal end of the catheter 104 is typically attached to a device, such as a syringe, that is manipulated to either inflate or deflate the balloon 102 by injecting a fluid into or withdrawing a fluid from, respectively, the interior volume of the balloon 102.

The conventional balloon catheter 100 has a number of drawbacks for use in many of the above-described procedures, and in particular, for use in PDT procedures. When initially manufactured, the balloon catheter 100 generally assumes a shape and configuration as depicted in FIG. 5A. As can be seen in this drawing, the central portion 110 of the balloon 102 is connected to the distal end 106 and the proximal end 108 by tapered or conical sections 116. The tapered sections 116 provide a transition between the larger diameter of the central portion 110 of the balloon 102 and the outermost portions of the balloon 102 (i.e., the distal end 106 and the proximal end 108) that are connected to the catheter 104.

At the time of packaging by the manufacturer or at the initiation of the medical procedure, the balloon 102 is typically deflated prior to inserting of the balloon catheter 100 into the body canal. Deflation of the balloon 102 is necessary to reduce the overall cross-section or diameter of the device to permit it to pass through an endoscope and/or to navigate and pass through the body's internal canals. FIG. 5B depicts the balloon catheter 100 in the deflated state. As can be seen in this drawing, the balloon 102 is forced to compress in length. This is because the overall length of the material that forms the central portion 110 and the tapered portions 116, as measured along the surface of the balloon 102 in a generally axial direction of the catheter 104 (i.e., from one end of the balloon 102 to the other), is greater than the distance between the distal end 106 and the proximal end 108. As a result of this compression, transverse creases 118 typically form along the surface of the balloon 102.

After the balloon catheter 100 is positioned within the body canal (not shown) at the desired location, inflation of the balloon 102 is initiated as shown in FIG. 5C. As depicted in this drawing, the creases 118 in the surface of the material may prevent the balloon 102 from fully expanding to its normal length (i.e., as shown in FIG. 5A). In other words, the balloon 102 tends to act like a spring under tension. As a result, the portion of the catheter 104 that lies between the distal end 106 and the proximal end 108 of the balloon 102 will be forced into compression, and may begin to bow 120 as a result of these compressive forces.

As inflation of the balloon 102 continues, bowing 120 of the catheter 104 may be increased as shown in FIG. 5D. This is the result of transverse or outward expansion of the central portion 110 of the balloon, which tends to pull the distal end 106 and the proximal end 108 towards each other.

Bowing 120 of the catheter 104 may not be eliminated until a sufficiently high inflation pressure is applied to the balloon 102 (see FIG. 5A). However, some bowing 120 of the catheter 104 may nevertheless remain if the initial deflation of the balloon 102 (see FIG. 5B) resulted in the formation of permanent transverse creases 118. Permanent bowing 120 of the catheter 104 is more likely if the balloon 102 is constructed from a non-elastomeric material.

The formation of transverse creases 118 and the bowing 120 of the catheter 104 can negatively impact the use of the conventional balloon catheter 100 during certain medical procedures. For example, during angioplasty procedures, permanent creases 118 in the surface of the balloon 102 may prevent the complete or uniform compression of the tissue on the interior surface of the body canal against which the balloon 102 is expanded. This may result in a decrease in effectiveness of the angioplasty procedure.

With respect to PDT procedures, any bowing 120 of the catheter 104 can prevent accurate alignment and centering of the catheter 104 within the body lumen or canal to be treated. This is because typical PDT procedures do not allow the expanded balloon 102 to exert excess pressure or heavy contact on the interior surface of the body lumen. Thus, the balloon 102 cannot be inflated with a pressure that is sufficient to eliminate any bowing 120 of the catheter 104. The catheter 104 may consequently not be properly centered in the body lumen. As a result, effective treatment of the body lumen tissue with the therapeutic fiber optic device, which is positioned inside the catheter 104, may be inhibited.

In addition, because the distal end 106 and the proximal end 108 of the balloon 102 are both fixed to the catheter 104 at permanent (i.e., non-moveable) locations, the ability to reduce the diameter of the deflated balloon 102 may be limited, particularly if the balloon 102 is manufactured from a non- elastomeric material. In other words, the central portion 110 of the balloon 102 may not compress tightly about the catheter 104 during deflation because of the creases 118 formed in the material of the balloon 102 (see FIG. 5B). Bunching of the balloon material may likewise limit the deflated diameter or cross-section of the balloon 102. Consequently, the device may be more difficult to maneuver during ingress or egress of the device through the body's canals. In addition, the resulting "wrinkled" surface of the balloon 102 may cause irritation to body canal tissue during ingress or egress of the device and/or prevent the device from passing through the endoscope channel.

What is needed is an improved balloon catheter that overcomes the disadvantages of the conventional devices. In particular, what is needed is a balloon catheter that can be deflated to a minimal diameter for ingress and egress through the body's canals and/or an endoscope channel, that resists the formation of transverse creases in the surface of the balloon during deflation, and that resists bowing of the catheter portion located within the balloon upon inflation.

Reference is directed to US 2003/0236495 which discloses arrangements of balloon catheters in which a slip joint is provided between one end of the balloon and a catheter or between segments of a two-piece catheter. This document discloses the features of the preamble of claim 1.

### SUMMARY OF THE INVENTION

The foregoing problems are solved and a technical advance is achieved by the balloon catheter of the present invention, which is defined in the appended claims. In various described examples, the balloon catheter includes a rounded or cylindrically shaped balloon that is affixed to a catheter. The balloon includes a distal end, a proximal end and a central portion, and may be formed of a non-elastomeric material. The balloon is attached to the catheter by inserting the distal end of the catheter into and through the proximal end of the balloon until the distal end of the catheter is inserted into a portion of the distal end of the balloon. The proximal end of the balloon is then affixed to the outer wall of the catheter so as to provide an air tight seal between these components.

The distal end of the catheter is not affixed to the distal end of the balloon. In one aspect of the invention, the catheter terminates at or near the proximal end of the balloon. A stiffening member is disposed within the catheter and extends distally through the interior of the balloon and forms a slip joint connection with the distal end of the balloon. The slip joint allows the distal end of the balloon to axially move or translate with respect to the distal end of the catheter while maintaining axial alignment of the balloon relative to the stiffening member.

The above-described configuration allows the overall length of the balloon to change during inflation or deflation, the change in length of the balloon not being impeded by the predetermined length of the catheter. In addition, the above-described configuration prevents the relative axial rigidity of the catheter and stiffening member from generating any axial tensile or compressive forces in the balloon. Consequently, transverse creasing of the central portion of the balloon is eliminated or at least minimized. Moreover, the central portion of the balloon can be collapsed into a smaller diameter or cross-section for ingress or egress of the balloon catheter through the body's canals and/or the endoscope channel.

The slip joint (or the elimination of a continuous catheter connected between both ends of the balloon) also prevents balloon from generating any adverse forces in the catheter during inflation or deflation of the device. In particular, since the distal end of the balloon is not rigidly connected to the distal end of the catheter, any axial contraction or expansion of the balloon will not impart any tensile or compressive forces along the axis of the catheter, and the catheter will not be bowed or stretched as result of the inflation or deflation of the balloon. Consequently, the catheter should remain centered with respect to cross-section of the balloon irrespective of the state of inflation of the balloon.

These and other advantages, as well as the invention itself, will become apparent in the details of construction and operation as more fully described below. Moreover, it should be appreciated that several aspects of the invention can be used with other types of balloon catheters or medical devices.

### BRIEF DESCRIPTION OF SEVERAL VIEWS OF THE DRAWINGS

Several illustrative examples and embodiments of the present invention will now be described by way of example with reference to the accompanying drawings, in which:
FIG. 1 is a cross-sectional side view of an illustrative example of a balloon catheter which is not falling under the scope of the invention;
FIG. 2 is a cross-sectional side view of a second example of a balloon catheter;
FIG. 3 is a cross-sectional side view of a third example of a balloon catheter;
FIG. 4 is a cross-sectional side view of a fourth example of a balloon catheter;
FIGS. 5A-5D depict cross-sectional side views of a conventional balloon catheter in various stages of inflation and deflation;
FIG. 6 is a cross-sectional side view of a fifth example of a balloon catheter;
FIG. 7 is a cross-sectional side view of a sixth example of a balloon catheter;
FIG. 8 is a cross-sectional side view of a first embodiment of a balloon catheter; and
FIG. 9 is a cross-sectional side view of a seventh example of a balloon catheter, not falling under the scope of the invention.

### DETAILED DESCRIPTION OF THE INVENTION

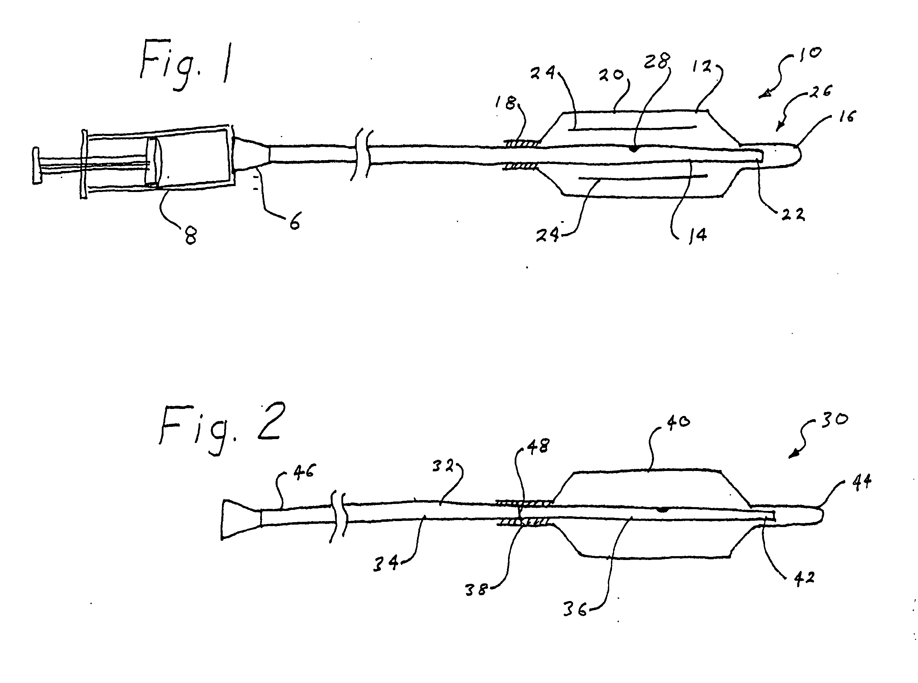

A first example of a balloon catheter 10 not forming part of the present invention is depicted in FIG. 1. The balloon catheter 10 includes a rounded, oval, cylindrical, bullet or other appropriately shaped balloon 12 that is affixed to a catheter 14. The balloon 12 is typically manufactured from a non-elastomeric material (e.g., a semi-rigid or non-compliant material), and preferably comprises a translucent, transparent or optically clear film. For example, the balloon 12 could be manufactured from a biocompatible polymer such as polyamide, polyurethane, polyester, polyolefin, polyethylene terephthalate and the like.

The balloon 12, as shown in the drawings, includes a distal end 16, a proximal end 18 and a central portion 20. However, different configurations or designs can also be utilized for the balloon 12. For example, the distal end 16 and the proximal end 18 could both comprise a tubular construction so as to form a neck. The balloon 12 is attached to the catheter 14 by inserting the distal end 22 of the catheter 14 into and through the proximal end 18 of the balloon 12. The balloon 12 is then slid over the catheter 14 until the distal end 22 of the catheter 14 is inserted into a portion of the distal end 16 of the balloon 12. The proximal end 18 of the balloon 12 is then affixed to the outer wall of the catheter 14 by an adhesive, ultrasonic welding, or some other method so as to anchor and seal the proximal end of the balloon 12. In the preferred embodiment shown, the inside diameter of the proximal end 18 is sized to fit tightly or snugly over the catheter 14 so as to improve the integrity of the seal between these two components.

The distal end 22 of the catheter 14 is not affixed to the distal end 16 of the balloon 12. As shown in the drawing, the distal end 22 of the catheter 14 extends partially, but not fully, into the distal end 16 of the balloon 12 so as to form a slip joint 26 between these two components. The slip joint 26 allows the distal end 16 of the balloon 12 to axially move or translate with respect to the distal end 22 of the catheter 14. This configuration allows the overall axial or longitudinal length of balloon 12 to change during inflation or deflation without transferring tensile or compressive forces to the catheter 14. For example, when the balloon 12 is deflated, the balloon 12 tends to elongate in the axial direction as the central portion 20 is drawn inwardly towards the catheter 14, thereby moving the distal end 16 of the balloon 12 distally from or relative to the distal end 22 of the catheter 14. Since the distal end 16 of the balloon 12 is not prevented from moving axially, transverse creasing of the central portion 20 of the balloon 12 during deflation is eliminated or at least minimized. Moreover, the central portion 20 of the balloon 12 can be collapsed into a smaller diameter or cross-section for ingress or egress of the balloon catheter 10 through the body's canals and/or the endoscope channel.

The slip joint 26 also prevents the application of adverse forces on the catheter 14 by the balloon 12 during inflation or deflation of the device. In particular, since the distal end 16 of the balloon 12 is not connected to the distal end 22 of the catheter 14, any axial contraction or expansion of the balloon 12 will not impart any tensile or compressive forces onto the catheter 14. In other words, the catheter 14 will not be bowed or stretched as result of the inflation or deflation of the balloon 12. Consequently, the catheter 14 should remain centered with respect to cross-sectional area of the balloon 12 irrespective of the state of inflation of the balloon 12.

By partially extending the distal end 22 of the catheter 14 into the distal end 16 of the balloon 12, the distal end 22 of the catheter 14 can provide some lateral or transverse support to the distal end 16 of the balloon 12. This lateral support can help to guide the device, and prevent the balloon 12 from folding or collapsing, as the device is being inserted into the body's canals. The length of the distal end 16 of the balloon 12, and the position of the distal end 22 of the catheter 14 therein, should be sufficient to permit these components to freely translate with respect to each other in response to all stages of inflation and deflation of the device.

The distal end 16 of the balloon 12 is sealed so as to enclose the balloon 12. In the preferred embodiment shown, the distal end 16 of the balloon 12 is formed by inserting and sealing a small rod into the neck of the balloon 12. The distal end 16 of the balloon 12 may also be rounded to improve the ingress of the balloon catheter 10 into and through the body's canals and lumens, as well as through the channel of an endoscope. In addition, the inside diameter of the distal end 16 of the balloon 12 is slightly larger than the outside diameter of the distal end 22 of the catheter 14 so as to permit air or fluid to enter or be removed from the interior volume of the balloon 12 by passing through the distal end 22 of the catheter 14. Alternatively, an aperture 28 may be provided in the wall of the catheter 14 at a location proximal to the distal end 22, but within the interior volume of the balloon 12.

The central portion 20 of the balloon 12 may be provided with longitudinally or axially extending pleats or folds 24. These folds 24 provide creases along which the surface of the balloon 12 will fold or pleat when deflated. The folds 24 permit the central portion 20 of the balloon 12 to be collapsed to a minimal cross-sectional area or diameter, and prevent the formation of transverse or lateral creases along the same area.

The proximal end 6 of the catheter 14 is typically connected to an inflation device 8, such as a standard medical syringe. The inflation device 8 is in fluid communication with the interior of the balloon 12 via a lumen extending through the inside of the catheter 14. The catheter 14 may also comprise additional lumens through which contrast fluids or guide wires (not shown) can be passed.

A second example of a balloon catheter 30 not forming part of the present invention is depicted in FIG. 2. The balloon catheter of this embodiment 30 is similar to the embodiment of the balloon catheter 10 shown in FIG. 1, but comprises a two-part catheter 32 having a relatively flexible portion 34 and a relatively rigid portion 36. The flexible portion 34 extends from approximately the proximal end 38 of the balloon 40 to the proximal end 46 of the catheter 32. The flexible portion 34 has a similar design and construction as that of the catheter 14 of the first embodiment shown in FIG. 1.

The rigid portion 36 extends from approximately the proximal end 38 of the balloon 40 to the distal end 42 of the catheter 32. In other words, the rigid portion 36 is that portion of the catheter 32 that is disposed within the balloon 40. The rigid portion 36 is less likely to sag under its own weight or the weight of the balloon 40, and may provide increased lateral support to the distal end 44 of the balloon 40. The increased rigidity of the rigid portion 36 of the catheter 32 may be particularly beneficial for use in PDT procedures, where proper centering and alignment of the therapeutic fiber optic device (not shown) within the catheter 32 is critical.

In the example shown, the flexible portion 34 is connected to the rigid portion 36 at a joint 48 that is preferably located within the proximal end 38 of the balloon 40. The proximal end 38 provides reinforcement to the joint 48, as well as improving the integrity of the seal between these components.

With the exception of the two-part catheter 32 described above, the remaining components of the balloon catheter 30 of the second embodiment are the same or similar to the components of the balloon catheter 10 of the first embodiment. A detailed description of these components and their functions will consequently not be repeated here.

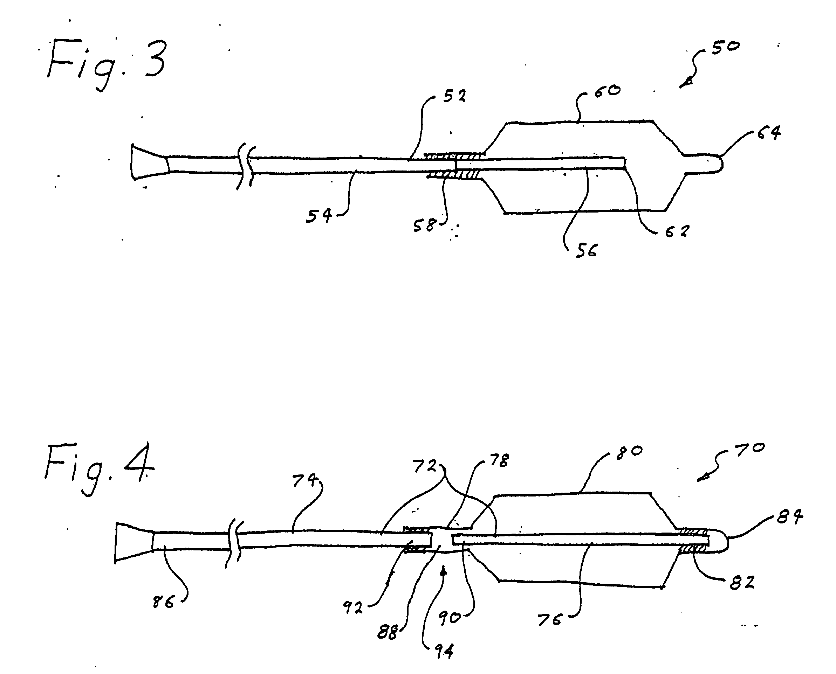

A third illustrative example of a balloon catheter 50 is depicted in FIG. 3. The balloon catheter 50 of this example is similar to the example of the balloon catheter 30 shown in FIG. 2 in that it also comprises a two-part catheter 52 having a flexible portion 54 and a rigid portion 56. However, the rigid portion 56 does not extend to the distal end 64 of the balloon 60. In other words, the rigid portion 56 only extends from near the proximal end 58 of the balloon 60 to part way into the interior volume of the balloon 60, and the distal end 62 of the rigid portion 56 does not form a slip joint with the distal end 64 of the balloon 60.

With the exception of the two-part catheter 52 described above, and the length of the rigid portion 56 thereof, the remaining components of the balloon catheter 50 of the third example are the same or similar to the components of the balloon catheter 30 of the second example. A detailed description of these components and their functions will consequently not be repeated here.

A fourth example of a balloon catheter 70 is depicted in FIG. 4. The balloon catheter of this example 70 is similar to the example of the balloon catheter 10 shown in FIG. 1, but comprises a segmented catheter 72 having a flexible portion 74 and a segmented or spaced apart portion 76. The flexible portion 74 extends from approximately the proximal end 78 of the balloon 80 to the proximal end 86 of the catheter 72. The flexible portion 74 has a similar design and construction as that of the catheter 14 of the first example shown in FIG. 1. The distal end 92 of the flexible portion 74 is affixed to the proximal end 78 of the balloon 80 by adhesive or some other form of bonding. The segmented portion 76 can be either rigid or flexible, and either hollow or solid. In other words, the segmented portion 76 can be a rod-like length of material as opposed to a catheter-like tube since the segmented portion 76 does not necessarily need to carry fluid between the inflation device (not shown) and the balloon 80.

The distal end 82 of the segmented portion 76 is affixed to the distal end 84 of the balloon 80. The segmented portion 76 extends proximally from the distal end 82 and terminates within the proximal end 78 of the balloon 80. The proximal end 90 of the segmented portion 76 is not affixed or bonded to the proximal end 78 of the balloon 80, but is free to move axially within the proximal end 78. In other words, a slip joint 94 is formed between the proximal end 90 of the segmented portion 76 and the proximal end 78 of the balloon 80. A gap 88 is provided between the proximal end 90 of segmented portion 76 and the distal end 92 of the flexible portion 74 within the proximal end 78 of the balloon 80. This gap 88 provides room for the segmented portion 76 to move longitudinally within the proximal end 78 of the balloon 80 as the balloon 80 longitudinally contracts or elongates during inflation and deflation, as well as allowing fluid from the inflation device (not shown) to pass through the distal end 92 of the flexible portion 74 and into the interior of the balloon 80. The proximal end 78 of the balloon 80 also provides lateral support to the proximal end 90 of the segmented portion 76.

This example has the advantage of allowing the balloon 80, and the segmented portion 76 of the catheter 72, to flex near the proximal end 78 of the balloon 80. This may provide increased maneuverability of the balloon catheter 70 during insertion of the device into and through the body's canals.

Of course, it should be appreciated that the segmented portion 76 could terminate short of the proximal end 78 of the balloon 80. In other words, the segmented portion 76 could extend only partially into the interior volume of the balloon 80, thereby eliminating any contact with the proximal end 78 of the balloon 80.

With the exception of the segmented catheter 72 described above, and the location of the slip joint 94 at the proximal end 78 of the balloon 80, the remaining components of the balloon catheter 70 of the fourth example are the same or similar to the components of the balloon catheter 10 of the first example. A detailed description of these components and their functions will consequently not be repeated here.

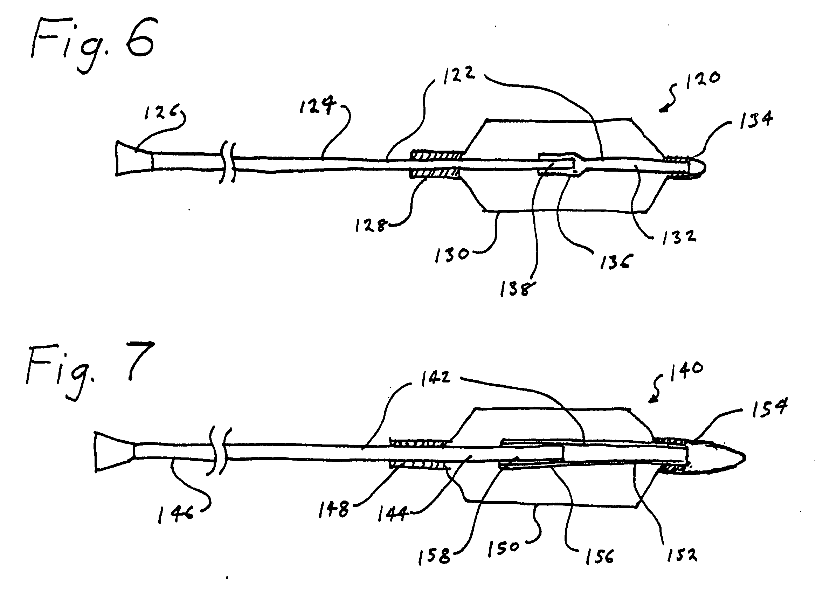

A fifth illustrative example of a balloon catheter 120 is depicted in FIG. 6. The balloon catheter of this example 120 is similar to the example of the balloon catheter 70 shown in FIG. 4 in that this example comprises a segmented or two-piece catheter 122. However, the proximal portion 124 of the catheter 122 extends from the proximal end 126 of the catheter, through the proximal end 128 of the balloon 130, and into the interior volume of the balloon 130 where it terminates near the mid-section of the balloon 130. The proximal portion 124 of the catheter 122 is affixed to the proximal end 128 of the balloon 130.

The distal portion 132 of the catheter 122 is affixed to the distal end 134 of the balloon 130, and likewise extends into the interior volume of the balloon 130 where it terminates near the mid-section of the balloon 130. The proximal end 136 of the distal portion 132 of the catheter 122 overlaps the distal end 138 of the proximal portion 124 of the catheter 122 in a sliding arrangement. In the example shown, the proximal end 136 of the distal portion 132 of the catheter 122 comprises an expanded tubular portion with an interior diameter that is slightly larger than the exterior diameter of the distal end 138 of the proximal portion 124 of the catheter 122 so as to permit relative axial movement between these two catheter components. This type of connection is often referred to as a male-female type of connection.

A sixth example of a balloon catheter 140 is depicted in FIG. 7. The balloon catheter of this example 140 is similar to the example of the balloon catheter 120 shown in FIG. 6 in that this example comprises a segmented or two-piece catheter 142, wherein the proximal portion 144 of the catheter 142 extends from the proximal end 146 of the catheter, through the proximal end 148 of the balloon 150, and into the interior volume of the balloon 150 where it terminates near the mid-section of the balloon 150. The proximal portion 144 of the catheter 142 is affixed to the proximal end 148 of the balloon 150.

The distal portion 152 of the catheter 142 is affixed to the distal end 154 of the balloon 150, and likewise extends into the interior volume of the balloon 150 where it terminates near the mid-section of the balloon 150. The proximal end 156 of the distal portion 152 of the catheter 142 overlaps the distal end 158 of the proximal portion 144 of the catheter 142 in a sliding arrangement. In the example shown, the distal portion 152 of the catheter 142 comprises a uniform tubular cross-section with an interior diameter that is slightly larger than the exterior diameter of the distal end 158 of the proximal portion 144 of the catheter 142 so as to permit relative axial movement between these two catheter components.

In the fifth and sixth examples (FIGS. 6 and 7), the overlapping portions of the separate catheter segments provide transverse or lateral stability to the balloon without impeding the axial expansion or contraction of the balloon. This is because the balloon is only fixedly connected to a either one of the catheter portions at single location.

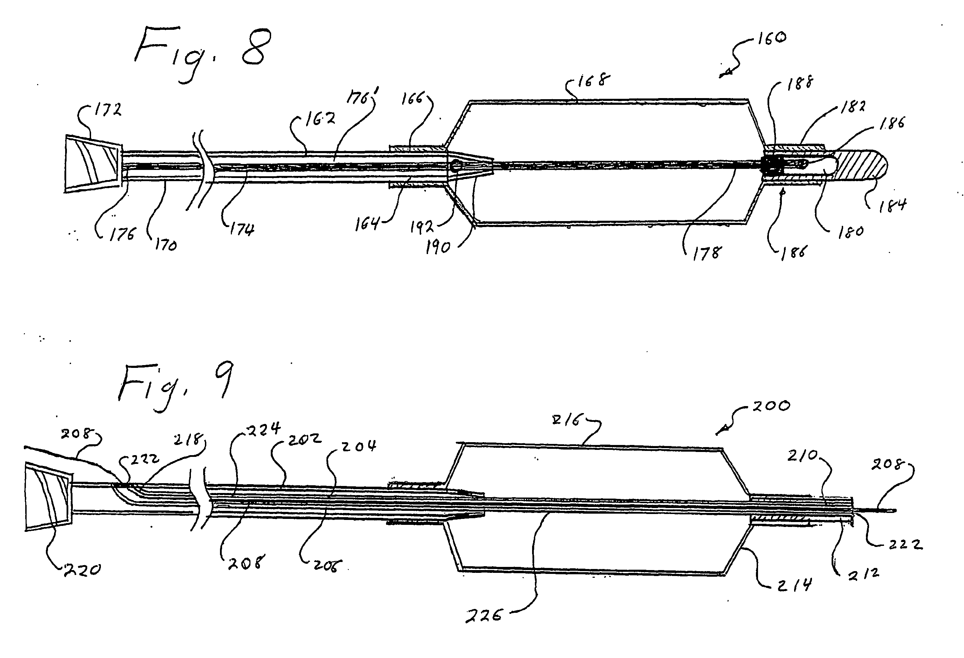

A embodiment of a balloon catheter 160 according to the present invention is depicted in FIG. 8. The balloon catheter 160 of this embodiment comprises a flexible elongate outer catheter 162 that is fixedly connected at its distal end 164 to the proximal end 166 of the balloon 168. The proximal end 170 of outer catheter 162 includes a luer fitting 172 that is configured to attach to an inflation device such a standard medical syringe (as shown in Fig. 1). The outer catheter 162 has a construction similar to that described in connection with the above embodiments.

The balloon catheter 160 further comprises an elongate stiffening member 174 disposed within the lumen 176' of the outer catheter 162. The diameter or cross-sectional area of the stiffening member 174 is generally less than the diameter or cross-sectional area of the lumen 176' so as to allow the passage of fluid between the luer fitting 172 (i.e., the inflation device) and the interior of the balloon 168. Alternatively, the outer catheter 162 may comprise a separate lumen for the passage of inflation fluid.

As illustrated in Fig. 8, the stiffening member 174 is connected at or near its proximal end 176 to the luer fitting 172. The distal end 178 of the stiffening member 174 extends distally from the distal end 164 of the outer catheter 162, through the interior of the balloon 168, and into a sleeve 180 formed in the distal end 182 of the balloon 168. In the embodiment shown, the sleeve 180 is formed by an end cap 184 fixed to the distal end 182 of the balloon 168. The end cap 184 provides an air tight seal with the balloon 168 and is rounded at its distal end to facilitate ingress of the balloon catheter 160 into and through the patient's bodily lumen and prevent the end cap 184 from puncturing or injuring the walls of the bodily lumen. The end cap 184 may be manufactured from a pliable plastic material to further promote the ingress of the balloon catheter 160 and reduce irritation that may be caused thereby.

The distal end 178 of the stiffening member 174 slidably engages with sleeve 180 to form a slip joint 186 that is similar to the slip joint 26 of the balloon catheter 10 shown in Fig. 1. As described in detail above, the slip joint 186 allows the distal end 182 of the balloon 168 to axially move or translate with respect to the distal end 178 of stiffening member 174. This configuration allows the overall axial or longitudinal length of balloon 168 to change during inflation or deflation without transferring tensile or compressive forces to either outer catheter 162 or stiffening member 174.

In the embodiment illustrated in Fig. 8, a collar or cannula 188 is disposed inside the sleeve 180. The cannula 188 has an inside diameter that is slightly greater than the outside diameter of stiffening member 174 so as to allow the stiffening member 174 to move axially or slide with respect to the cannula 188. In other words, slip joint 186 is formed by the interaction of stiffening member 174 with cannula 188. The cannula 188 aligns stiffening member 174 with the central axis of the distal end 182 of the balloon 168. A press-fit connection is utilized to dispose cannula 188 within the sleeve 180 of end cap 184. The press-fit connection is formed by manufacturing the cannula 188 to have an outside diameter that is slightly larger than the inside diameter of sleeve 180. The cannula 188 may be comprised of metal or other radiopaque material so as to provide a radiopaque reference point for accurately positioning the distal end 182 of the balloon 168 within the patient.

The distal end 178 of stiffening member 174 comprises a bead 186. The bead 186 has a rounded tip to reduce friction between the distal end 178 of stiffening member 174 and the inside surface of the sleeve 180 of end cap 184, particularly if end cap 184 has been curved by the process of inserting balloon catheter 160 into the patient's bodily lumen. The bead 186 also comprises a cross-sectional diameter that is larger than the inside diameter of cannula 188. This arrangement prevents the distal end 182 of the balloon 168 from disconnecting from the stiffening member 174 in the event that balloon 168 should rupture within the patient. More specifically, if the distal end 182 of the balloon 168 becomes separated from the remainder of the balloon 168, the bead 186 will prevent the cannula 188 from sliding off the distal end 178 of the stiffening member 174.

In the embodiment illustrated in Fig. 8, stiffening member 174 is fixedly engaged with the distal end 164 of the outer catheter 162. More specifically, the distal end 164 of the outer catheter 162 comprises a tapered guide member 190 that reduces the interior diameter of the lumen 176 of outer catheter 162 down to the outer diameter of the stiffening member 174. Alternatively, guide member 190 may be configured to permit a sliding engagement between stiffening member 174 and the distal end 164 of the outer catheter 162. The reduced diameter of the distal end of the guide member 190 aligns the stiffening member 174 with the central axis of the proximal end 166 of the balloon 168. The guide member 190 comprises one or more openings or ports 192 to allow the passage of inflation fluid between the lumen 176 of the outer catheter 162 and the interior of the balloon 168. The guide member 190 may be comprised of metal or other radiopaque material so as to provide a radiopaque reference point for accurately positioning the proximal end 166 of the balloon 168 within the patient.

Stiffening member 174 comprises a solid wire that may have a stiffness or resistance to bending that is greater than the stiffness or resistance to bending of the outer catheter 162. In addition to maintaining alignment of the distal end 186 of the balloon 168, the stiffening member 174 enhances the overall stiffness and pushability of balloon catheter 160. In other words, overall stiffness and pushability of balloon catheter 160 is achieved by the combination of the stiffening member 174 and the outer catheter 162. The stiffening member 174 may also provide a radiopaque reference line for accurately positioning the central axis (or centerline) of the balloon 168 within the patient.

The stiffening member 174 may have either a circular or non-circular cross-section. In particular, a non-circular cross-section (e.g., triangular or star-shaped) may be utilized to increase the strength or stiffness of the stiffening member 174 without inhibiting the flow of inflation fluid through the lumen 176 of the outer catheter 162. The stiffening member 174 may also comprise hollow cross-section with a lumen disposed therein. As will be explained below in connection with the eighth embodiment shown in Fig. 9, a lumen extending through the stiffening member 174 could be used to accommodate a wire guide.

The stiffening member 174 may have non-uniform properties along the length thereof. For example, the stiffening member 174 may be tapered (e.g., having a decreasing cross-section) so as to have stiffness that decreases from its proximal end 176 to its distal end 178. The stiffening member 174 may also be manufactured from different materials having different physical properties. A stiffening member 174 having a decreasing stiffness along the length thereof would provide the balloon catheter 160 with greater stiffness near the proximal end 170 where the ability to push the balloon catheter 200 (i.e., "pushability") is most important, while providing greater flexibility near the distal end 164 where the ability to guide the balloon catheter 200 around tortuous pathways is most important. The stiffening member 174 may also be manufactured from different materials having different physical properties.

A seventh illustrative example of a balloon catheter 200 not forming part of the present invention is depicted in FIG. 9. The balloon catheter 200 of this example is similar to the embodiment of the balloon catheter 160 shown in FIG. 8 in that this example comprises an outer catheter 202 and separate inner member 204 disposed therein. However, in the balloon catheter 200 of this example, inner member 204 comprises a cannula or catheter having an inner lumen 206 adapted to receive a wire guide 208. The distal end 210 of inner member 204 is bonded to end cap 212 at the distal end 214 of the balloon 216. The proximal end 218 of inner member 204 passes through or is attached to the wall of outer catheter 202 near luer fitting 220. Ports 222 are provided at each end 210, 218 of inner member 204 to provide access for the wire guide 208 into and out of the inner lumen 206. Other features of this example are similar to the other embodiments described above and need not be repeated here.

In this example, outer catheter 202 and inner member 204 are each fixedly connected to balloon 216 and to each other. As a consequence, outer catheter 202 and/or inner member 204 are configured to accommodate any lengthening or shortening of the balloon 216 caused by the inflation or deflation thereof. In other words, balloon catheter 200 is configured so that the length of outer catheter 202 and/or inner member 204 will respond to and accommodate any changes in the length of the balloon 216. Because the overall length of outer catheter 202 and inner member 204 is much greater than the length of balloon 216, the total axial expansion or contraction that must be accommodated by the outer catheter 202 and/or inner member 204 is spread out over a relatively long distance as incrementally much smaller than that of the balloon 216.

In the example illustrated in Fig. 9, inner member 204 comprises a proximal section 224 and a distal section 226 having different physical or material properties. The proximal section 224 is constructed of a flexible material or otherwise configured to readily expand or contract in length (relative to outer member 202) in response to changes in the length of the balloon 216. In contrast to the proximal section 224, the distal section 226 is constructed of a relatively rigid material so as to provide lateral support to the distal end 214 of the balloon 216. The more rigid distal section 226 may also extend proximally of the balloon 216 to provide additional stiffness to the outer catheter 202. The portion the outer catheter 202 adjacent to the more flexible proximal section 224 of the inner member 204 may be stiffened to avoid weak areas that may be prone kinking. Stiffening of the outer catheter 202 can be accomplished by, for example, increasing the cross-sectional area of the outer catheter 202 or including a separate stiffening member (not shown).

Alternatively, outer catheter 202 may comprise a material of increased elasticity that will readily elongate in response changes in the length of the balloon 216. For example, the outer catheter 202 (or a portion thereof) may be constructed of a flexible material or otherwise configured to readily expand or contract in length (relative to inner member 204) in response changes in the length of the balloon 216. In such an example, the inner member 204 may be constructed of a relatively rigid material so as to provide stiffness or lateral support to the outer catheter 202 as well as to the distal end 214 of the balloon 216. In other words, the inner member 204 will act as the primary stiffening member for balloon catheter 200.

The balloon catheter 200 of this example is adapted for use in medical procedures wherein a wire guide 208 is pre-positioned in the patient's bodily lumen. In such a procedure, the proximal end of wire guide 208, which extends outside of the patient, is inserted through port 222 and into inner lumen 208 of distal end of balloon catheter 200 (i.e., into the distal end 212 of inner member 206). The balloon catheter 200 is then pushed over the wire guide 208 until the balloon 216 is positioned at the desire location within the patient. The wire guide 208, which may have been previously positioned within the patient during an earlier part of the medical procedure, allows the balloon catheter 200 to be quickly inserted and guided into the patient. The balloon 216 is then inflated as described above in connection with the other embodiments.

It should be appreciated that the wire guide 208 must be long enough so that the portion of the wire guide 208 extending out of the patient is longer than the overall length of the inner member 204 of the balloon catheter 200. This length is necessary so that the proximal end of wire guide 208 will extend out of inner lumen 208 at the proximal end 218 (and proximal port 222) of inner member 204 prior to the distal end 210 of inner member 204 (or end cap 212) is inserted into the patient. This allows the wire guide 208 to be grasped and held in position at all times while the balloon catheter 200 is being fed onto the wire guide 208 and inserted into the patient.

In the example illustrated in Fig. 9, the proximal end 218 (and proximal port 222) of inner member 204 is located relatively near connector 220. Inner member 204 therefore extends along a substantial portion of balloon catheter 200. Catheter devices having a wire guide lumen extending along substantially the entire overall length of the device are commonly referred to as over-the-wire devices. However, a shorter inner member 204 could be utilized. For example, the proximal end 218 (and proximal port 222) of inner member 204 could be located much closer to the balloon 216. Alternatively, additional ports 222 could be provided along the outer catheter to give access to the inner lumen 208 at intermediate locations. In such embodiments, the wire guide 208 would exit inner lumen 208 at a location near the proximal end of the balloon 216. The arrangement requires a much shorter portion of the wire guide 208 to extend out of the patient since the length of the inner lumen 208 is much shorter than the length of the balloon catheter 200. Catheter devices having a shorter wire guide lumen, or having intermediate access to the wire guide lumen, are commonly referred to as rapid exchange devices.

Any other undisclosed or incidental details of the construction or composition of the various elements of the disclosed embodiments of the present invention are not considered to be critical to the achievement of the advantages of the present invention, so long as the elements possess the attributes required to perform as disclosed herein. The selection of these and other details of construction are believed to be well within the ability of one of ordinary skill in the relevant art in view of the present disclosure. Illustrative embodiments of the present invention have been described in considerable detail for the purpose of disclosing practical, operative structures whereby the invention may be practiced advantageously. The designs described herein are intended to be exemplary only. The novel characteristics of the invention may be incorporated in other structural forms without departing from the scope of the invention, as set forth in the appended claims.

## Claims

1. A balloon catheter (160) comprising:
an inflatable balloon (168) comprising a balloon wall defining an interior volume, the balloon further comprising a distal end (182), a proximal end (166), and a central portion disposed therebetween;
a catheter (162) comprising an elongated shaft extending along an axis between a distal end portion (164) and a proximal end portion (170), the proximal end portion comprising a connector (172) configured to engage an inflation device, the distal end portion (164) fixedly connected to the proximal end (166) of the balloon (168), and a lumen extending through the shaft and in fluid communication with the interior volume of the balloon; and
a stiffening member (174) comprising a proximal portion (176) and a distal portion (178), the balloon catheter (160) being **characterized in that** the proximal portion (176) of said stiffening member (174) being disposed within the lumen (176') of the shaft and having a cross-sectional area that is less than a cross-sectional area of the lumen (176') so as to permit the passage of a fluid between the lumen and the stiffening member, the distal portion (178) extending distally from the distal end portion (164) of the catheter and through the interior volume of the balloon, the stiffening member being non-fixedly connected to the distal end of the balloon, wherein movement of the distal end (182) of the balloon relative to the proximal end (166) of the balloon is not restrained by the catheter (162), wherein axial movement of the distal end (182) of the balloon relative to the proximal end (166) of the balloon in a direction generally parallel to the axis of the shaft is not restrained by the stiffening member (174), and wherein transverse movement of the distal end (182) of the balloon relative to the proximal end (166) of the balloon in a direction generally perpendicular to the axis of the shaft is restrained by the stiffening member (174).

2. The balloon catheter according to claim 1 wherein the balloon has a deflated axial length when deflated, and an inflated axial length when inflated, the deflated axial length and the inflated axial length each being defined by the distance between the proximal end and the distal end of the balloon, the deflated axial length being different than the inflated axial length.

3. The balloon catheter according to claim 1 wherein the balloon has a deflated axial length when deflated, and a partially inflated axial length when partially inflated, the deflated axial length and the partially inflated axial length each being defined by the distance between the proximal end and the distal end of the balloon, the deflated axial length being different than the partially inflated axial length.

4. The balloon catheter according to claim 1 wherein the balloon has a partially inflated axial length when partially inflated, and a fully inflated axial length when fully inflated, the partially inflated axial length and the fully inflated axial length each being defined by the distance between the proximal end and the distal end of the balloon, the partially inflated axial length being different than the fully inflated axial length.

5. The balloon catheter according to claim 1 wherein the balloon wall comprises one of a non-elastic material, a non-compliant material, and a semi-rigid material.

6. The balloon catheter according to claim 1 wherein the balloon wall comprises axially oriented creases or pleats to facilitate radial compression of the balloon when deflated.

7. The balloon catheter according to claim 1 wherein the proximal portion of the stiffening member extends along and generally parallel to the shaft of the catheter.

8. The balloon catheter according to claim 7 wherein a proximal end of the proximal portion of the stiffening member is fixedly connected to the proximal end portion of the catheter.

9. The balloon catheter according to claim 1 wherein the distal end portion of the catheter comprises a distal end that terminates within the interior volume of the balloon, the distal end comprising a port to permit the inflation fluid to flow between the lumen of the catheter and the interior volume of the balloon.

10. The balloon catheter according to claim 1 wherein the distal end portion of the catheter comprises a distal end that terminates within the interior volume of the balloon, the distal end being fixedly connected to the stiffening member.

11. The balloon catheter according to claim 1 wherein the distal end portion of the catheter comprises a distal end that terminates within the interior volume of the balloon, the distal end being in sliding engagement with the stiffening member so as to align the stiffening member with the axis of the shaft and prevent transverse movement of the stiffening member in a direction generally perpend icular to the axis of the shaft.

12. The balloon catheter according to claim 1 wherein the distal end of the balloon comprises a sleeve, a distal end of the stiffening member being slidably disposed within the sleeve.

13. The balloon catheter according to claim 12 wherein the sleeve comprises a distal terminus that is spaced away from the distal end of the stiffening member so as to permit axial movement of the distal end of the stiffening member relative to the distal terminus of the sleeve.

14. The balloon catheter according to claim 12 wherein the sleeve comprises a cannula disposed therein, the cannula having an interior cross- sectional area that is less than an interior cross-sectional area of the sleeve, the interior cross-sectional area of the cannula configured to slidingly engage an exterior surface of the sleeve.

15. The balloon catheter according to claim 14 wherein the distal end of the stiffening member comprises a retaining portion, the retaining portion having an exterior cross-sectional area that is greater than the interior cross-sectional area of the cannula so as to prevent the distal end of the stiffening member from passing through the canula.

16. The balloon catheter according to claim 15 wherein the retaining portion comprises a rounded bead affixed to the distal end of the stiffening member, the bead having a diameter that is greater than an inside diameter of the cannula.

17. The balloon catheter according to claim 12 wherein the distal end of the balloon comprises an end cap affixed thereto, the sleeve being defined by an interior volume of the end cap.

18. The balloon catheter according to claim 1 further comprising an inflation device for inflating or deflating said balloon, said inflation device being attached to the connector on the proximal end portion of the catheter.

19. The balloon catheter according to claim 18 wherein the connector comprises a female luer fitting, and further wherein the inflation device comprises a syringe having a male luer fitting, the mate luer fitting being engaged with the female luer fitting.

20. The balloon catheter according to claim 8 wherein the stiffening member comprises a solid wire having a circular cross-section.

21. The balloon catheter according to claim 8 wherein the stiffening member comprises a lumen configured to accommodate the passage of a wire guide.

22. The balloon catheter according to claim 8 wherein the stiffening member has a first physical property at a first location and a second physical property at a second location, the first physical property being different than the second physical property.

23. The balloon catheter according to claim 22 wherein the first physical property comprises a first stiffness and the second physical property comprises a second stiffness, the first stiffness being greater than the second stiffness, and the first location being proximal of the second location.

24. The balloon catheter according to claim 22 wherein the stiffening member has a tapered cross-section.

## Patentansprüche

1. Ballonkatheter (160), der Folgendes umfasst:
einen aufblasbaren Ballon (168) mit einer Ballonwand, die ein Innenlumen definiert, wobei der Ballon ferner ein distales Ende (182), ein proximales Ende (166) und einen dazwischen angeordneten zentralen Abschnitt umfasst; ein Katheter (162) mit einem länglichen Schaft, der sich entlang einer Achse zwischen einem distalen Endabschnitt (164) und einem proximalen Endabschnitt (170) erstreckt, wobei der proximale Endabschnitt ein Verbindungsglied (172), das in eine Aufblasvorrichtung eingreifen kann, wobei der distale Endabschnitt (164) fest mit dem proximalen Ende (166) des Ballons (168) verbunden ist, sowie ein Lumen umfasst, das sich durch den Schaft erstreckt und mit dem Innenvolumen des Ballons in Fluidverbindung steht;
und ein Versteifungselement (174) mit einem proximalen Abschnitt (176) und einem distalen Abschnitt (178),
wobei der Ballonkatheter (160) **dadurch gekennzeichnet ist, dass** der proximale Abschnitt (176) des Versteifungselements (174) im Lumen (176') des Schafts angeordnet ist und eine Querschnittsfläche aufweist,
die kleiner ist als die Querschnittsfläche des Lumens (176') um den Durchgang einer Flüssigkeit zwischen dem Lumen und dem Versteifungselement zu gestatten, wobei sich der distale Abschnitt (178) distal vom distalen Endabschnitt (164) des Katheters und durch das Innenvolumen des Ballons erstreckt, wobei das Versteifungselement nicht fest mit dem distalen Ende des Ballons verbunden ist, worin eine Bewegung des distalen Endes (182) des Ballons relativ zum proximalen Ende (166) des Ballons durch den Katheter (162) nicht unterdrückt wird, worin ein axiale Bewegung des distalen Endes (182) des Ballons relativ zum proximalen Ende (166) des Ballons in einer zur Achse des Schafts allgemein parallelen Richtung vom Versteifungselement (174) nicht unterdrückt wird, und worin eine Querbewegung des distalen Endes (182) des Ballons relativ zum proximalen Ende (166) des Ballons in einer zur Achse des Schafts allgemein lotrechten Richtung vom Versteifungselement (174) unterdrückt wird.

2. Ballonkatheter nach Anspruch 1, worin der Ballon nach dem Entleeren eine entleerte Axiallänge und beim Aufblasen eine aufgeblasene Axiallänge aufweist, wobei die entleerte Axiallänge und die aufgeblasene Axiallänge jeweils durch den Abstand zwischen dem proximalen Ende und dem distalen Ende des Ballons bestimmt werden, wobei sich die entleerte Axiallänge von der aufgeblasenen Axiallänge unterscheidet.

3. Ballonkatheter nach Anspruch 1, worin der Ballon nach dem Entleeren eine entleerte Axiallänge und beim teilweisen Aufblasen eine teilweise aufgeblasene Axiallänge aufweist, wobei die entleerte Axiallänge und die teilweise aufgeblasene Axiallänge jeweils vom Abstand zwischen dem proximalen Ende und dem distalen Ende des Ballons definiert werden, wobei sich die entleerte Axiallänge von der teilweise aufgeblasenen Axiallänge unterscheidet.

4. Ballonkatheter nach Anspruch 1, worin der Ballon beim teilweisen Aufblasen eine teilweise aufgeblasene Axiallänge und beim vollständigen Aufblasen eine vollständig aufgeblasene Axiallänge aufweist, wobei die teilweise entleerte Axiallänge und die vollständig aufgeblasene Axiallänge jeweils vom Abstand zwischen dem proximalen Ende und dem distalen Ende des Ballons definiert werden, wobei sich die teilweise aufgeblasene Axiallänge von der vollständig aufgeblasenen Axiallänge unterscheidet.

5. Ballonkatheter nach Anspruch 1, worin die Ballonwand aus einem nicht-elastischen Material, einem nicht-nachgiebigen Material oder einem halbstarren Material besteht.

6. Ballonkatheter nach Anspruch 1, worin die Ballonwand axial orientierte Knickfalten oder Falten umfasst, um die radiale Kompression des entleerten Ballons zu erleichtern.

7. Ballonkatheter nach Anspruch 1, worin der proximale Abschnitt des Versteifungselements sich über den und im Allgemeinen parallel zum Schaft des Katheters erstreckt.

8. Ballonkatheter nach Anspruch 7, worin ein proximales Ende des proximalen Abschnitts des Versteifungselements fest mit dem proximalen Endabschnitt des Katheters verbunden ist.

9. Ballonkatheter nach Anspruch 1, worin der distale Endabschnitt des Katheters ein distales Ende umfasst, das im Innenvolumen des Ballons endet, wobei das distale Ende eine Öffnung umfasst, damit die Aufblasflüssigkeit zwischen dem Lumen des Katheters und dem Innenvolumen des Ballons fließen kann.

10. Ballonkatheter nach Anspruch 1, worin der distale Endabschnitt des Katheters ein distales Ende umfasst, das im Innenvolumen des Ballons endet, wobei das distale Ende fest mit dem Versteifungselement verbunden ist.

11. Ballonkatheter nach Anspruch 1, worin der distale Endabschnitt des Katheters ein distales Ende umfasst, das im Innenvolumen des Ballons endet, wobei sich das distale Ende in gleitendem Eingriff mit der Versteifungselement befindet, um das Versteifungselement mit der Achse des Schafts auszurichten und eine Querbewegung des Versteifungselements in einer zur Achse des Schafts allgemein lotrechten Richtung zu verhindern.

12. Ballonkatheter nach Anspruch 1, worin das distale Ende des Ballons eine Hülse umfasst, wobei ein distales Ende des Versteifungselements gleitend in der Hülse angeordnet ist.

13. Ballonkatheter nach Anspruch 12, worin die Hülse ein distales Ende umfasst, das vom distalen Ende des Versteifungselements beabstandet ist, um eine Axialbewegung des distalen Endes des Versteifungselements relativ zum distalen Ende der Hülse zu gestatten.

14. Ballonkatheter nach Anspruch 12, worin die Hülse eine darin angeordnete Kanüle umfasst, wobei die Kanüle eine innere Querschnittsfläche aufweist, die kleiner ist als eine innere Querschnittsfläche der Hülse, wobei die innere Querschnittsfläche der Kanüle für gleitenden Eingriff in eine Außenseite der Hülse konfiguriert ist.

15. Ballonkatheter nach Anspruch 14, worin das distale Ende des Versteifungselements einen Halteabschnitt umfasst, wobei der Halteabschnitt eine äußere Querschnittsfläche aufweist, die größer ist als die innere Querschnittsfläche der Kanüle, um zu verhindern, dass das distale Ende des Versteifungselements durch die Kanüle läuft.

16. Ballonkatheter nach Anspruch 15, worin der Halteabschnitt einen abgerundeten Wulst umfasst, der am distalen Ende des Versteifungselements befestigt ist, wobei der Wulst einen Durchmesser aufweist, der größer ist als ein Innendurchmesser der Kanüle.

17. Ballonkatheter nach Anspruch 12, worin das distale Ende des Ballons eine daran befestigte Endkappe umfasst, wobei die Hülse von einem Innenvolumen der Endkappe definiert wird.

18. Ballonkatheter nach Anspruch 1, der ferner eine Aufblasvorrichtung zum Aufblasen und Entleeren des Ballons umfasst, wobei die Aufblasvorrichtung mit dem Verbindungsglied am proximalen Endabschnitt des Katheters befestigt ist.

19. Ballonkatheter nach Anspruch 18, worin das Verbindungsglied einen weiblichen Luer-Anschluss umfasst, und worin ferner die Aufblasvorrichtung eine Spritze mit einem männlichen Luer-Anschluss umfasst, wobei der männliche Luer-Anschluss mit dem weiblichen Luer-Anschluss in Eingriff steht.

20. Ballonkatheter nach Anspruch 8, worin das Versteifungselement einen festen Draht mit einem kreisförmigen Querschnitt umfasst.

21. Ballonkatheter nach Anspruch 8, worin das Versteifungselement ein Lumen umfasst, dass so konfiguriert ist, dass ein Führungsdraht durch es hindurch geführt werden kann.

22. Ballonkatheter nach Anspruch 8, worin das Versteifungselement eine erste physikalische Eigenschaft an einer ersten Stelle und eine zweite physikalische Eigenschaft an einer zweiten Stelle aufweist, wobei die erste physikalische Eigenschaft sich von der zweiten physikalischen Eigenschaft unterscheidet.

23. Ballonkatheter nach Anspruch 22, worin die erste physikalische Eigenschaft eine erste Steifheit umfasst und die zweite physikalische Eigenschaft eine zweite Steifheit umfasst, wobei die erste Steifheit größer ist als die zweite Steifheit, und wobei die erste Stelle proximal zur zweiten Stelle liegt.

24. Ballonkatheter nach Anspruch 22, worin das Versteifungselement einen verjüngten Querschnitt aufweist.

## Revendications

1. Sonde (160) à ballonnet comportant :
un ballonnet (168) gonflable comportant une paroi de ballonnet définissant un volume intérieur, le ballonnet comportant en outre une extrémité (182) distale, une extrémité (166) proximale, et une portion centrale disposée entre les deux extrémités ;
une sonde (162) comportant une tige allongée s'étendant le long d'un axe entre une portion (164) d'extrémité distale et une portion (170) d'extrémité proximale, la portion d'extrémité proximale comportant un connecteur (172) conçu pour se mettre en prise avec un dispositif de gonflage, la portion (164) d'extrémité distale étant reliée de manière fixe à l'extrémité (166) proximale du ballonnet (168), et une lumière s'étendant à travers la tige et en communication fluidique avec le volume intérieur du ballonnet ; et
un organe (174) de raidissement comportant une portion (176) proximale et une portion (178) distale, la sonde (160) à ballonnet étant **caractérisée en ce que** la portion (176) proximale dudit organe (174) de raidissement est disposée à l'intérieur de la lumière (176') de la tige et présente une zone de section transversale qui est inférieure à une zone de section transversale de la lumière (176') de façon à ce qu'un fluide puisse passer entre la lumière et l'organe de raidissement, la portion (178) distale s'étendant distalement par rapport à la portion (164) d'extrémité distale de la sonde et à travers le volume intérieur du ballonnet, l'organe de raidissement étant relié de manière non fixe à l'extrémité distale du ballonnet, dans laquelle le déplacement de l'extrémité (182) distale du ballonnet par rapport à l'extrémité (166) proximale du ballonnet n'est pas limitée par la sonde (162), dans laquelle le déplacement axial de l'extrémité (182) distale du ballonnet par rapport à l'extrémité (166) proximale du ballonnet dans une direction en général parallèle à l'axe de la tige n'est pas limité par l'organe (174) de raidissement, et dans laquelle le déplacement transversal de l'extrémité (182) distale du ballonnet par rapport à l'extrémité (166) proximale du ballonnet dans une direction en général perpendiculaire à l'axe de la tige est limité par l'organe (174) de raidissement.

2. Sonde à ballonnet selon la revendication 1 dans laquelle le ballonnet a une longueur axiale dégonflée lorsqu'il est dégonflé, et une longueur axiale gonflée lorsqu'il est gonflé, la longueur axiale dégonflée et la longueur axiale gonflée étant chacune définie par la distance entre l'extrémité proximale et l'extrémité distale du ballonnet, la longueur axiale dégonflée étant différente de la longueur axiale gonflée.

3. Sonde à ballonnet selon la revendication 1 dans laquelle le ballonnet a une longueur axiale dégonflée lorsqu'il est dégonflé, et une longueur axiale partiellement gonflée lorsqu'il est partiellement gonflé, la longueur axiale dégonflée et la longueur axiale partiellement gonflée étant chacune définie par la distance entre l'extrémité proximale et l'extrémité distale du ballonnet, la longueur axiale dégonflée étant différente de la longueur axiale partiellement gonflée.

4. Sonde à ballonnet selon la revendication 1 dans laquelle le ballonnet a une longueur axiale partiellement gonflée lorsqu'il est partiellement gonflé, et une longueur axiale totalement gonflée lorsqu'il est totalement gonflé, la longueur axiale partiellement gonflée et la longueur axiale totalement gonflée étant chacune définie par la distance entre l'extrémité proximale et l'extrémité distale du ballonnet, la longueur axiale partiellement gonflée étant différente de la longueur axiale totalement gonflée.

5. Sonde à ballonnet selon la revendication 1 dans laquelle la paroi du ballonnet comporte un matériau non élastique, un matériau non adaptif, et/ou un matériau semi-rigide.

6. Sonde à ballonnet selon la revendication 1 dans laquelle la paroi de ballonnet comporte des plis ou replis orientés axialement pour faciliter la compression radiale du ballonnet lorsqu'il est dégonflé.

7. Sonde à ballonnet selon la revendication 1 dans laquelle la portion proximale de l'organe de raidissement s'étend le long de la tige et en général parallèlement à la tige de la sonde.

8. Sonde à ballonnet selon la revendication 7 dans laquelle une extrémité proximale de la portion proximale de l'organe de raidissement est reliée de manière fixe à la portion d'extrémité proximale de la sonde.

9. Sonde à ballonnet selon la revendication 1 dans laquelle la portion d'extrémité distale de la sonde comporte une extrémité distale qui se termine à l'intérieur du volume intérieur du ballonnet, l'extrémité distale comportant un orifice pour permettre au fluide de gonflage de s'écouler entre la lumière de la sonde et le volume intérieur du ballonnet.

10. Sonde à ballonnet selon la revendication 1 dans laquelle la portion d'extrémité distale de la sonde comporte une extrémité distale qui se termine à l'intérieur du volume intérieur du ballonnet, l'extrémité distale étant reliée de manière fixe à l'organe de raidissement.

11. Sonde à ballonnet selon la revendication 1 dans laquelle la portion d'extrémité distale de la sonde comporte une extrémité distale qui se termine à l'intérieur du volume intérieur du ballonnet, l'extrémité distale étant en prise de manière coulissante avec l'organe de raidissement de façon à aligner l'organe de raidissement sur l'axe de la tige et empêcher un déplacement transversal de l'organe de raidissement dans un sens en général perpendiculaire à l'axe de la tige.

12. Sonde à ballonnet selon la revendication 1 dans laquelle l'extrémité distale du ballonnet comporte un manchon, une extrémité distale de l'organe de raidissement étant disposée de manière coulissante à l'intérieur du manchon.

13. Sonde à ballonnet selon la revendication 12 dans laquelle le manchon comporte une terminaison distale qui est éloignée de l'extrémité distale de l'organe de raidissement de façon à permettre un déplacement axial de l'extrémité distale de l'organe de raidissement par rapport à la terminaison distale du manchon.

14. Sonde à ballonnet selon la revendication 12 dans laquelle le manchon comporte une canule disposée à l'intérieur de celui-ci, la canule présentant une zone de section transversale intérieure plus petite que la zone de section transversale intérieure du manchon, la zone de section transversale intérieure de la canule étant conçue pour se mettre en prise de manière coulissante avec une surface extérieure du manchon.

15. Sonde à ballonnet selon la revendication 14 dans laquelle l'extrémité distale de l'organe de raidissement comporte une portion de retenue, la portion de retenue présentant une zone de section transversale extérieure plus grande que la zone de section transversale intérieure de la canule de façon à empêcher l'extrémité distale de l'organe de raidissement de passer dans la canule.

16. Sonde à ballonnet selon la revendication 15 dans laquelle la portion de retenue comporte une perle arrondie placée sur l'extrémité distale de l'organe de raidissement, la perle ayant un diamètre qui est plus grand que le diamètre interne de la canule.

17. Sonde à ballonnet selon la revendication 12 dans laquelle l'extrémité distale du ballonnet comporte un capuchon d'extrémité placé sur celui-ci, le manchon étant défini par un volume intérieur du capuchon d'extrémité.

18. Sonde à ballonnet selon la revendication 1 comportant en outre un dispositif de gonflage pour gonfler ou dégonfler ledit ballonnet, ledit dispositif de gonflage étant attaché au connecteur sur la portion d'extrémité proximale de la sonde.

19. Sonde à ballonnet selon la revendication 18 dans laquelle le connecteur comporte un raccord Luer femelle, et en outre dans laquelle le dispositif de gonflage comporte une seringue munie d'un raccord Luer mâle, le raccord Luer mâle étant mis en prise avec le raccord Luer femelle.

20. Sonde à ballonnet selon la revendication 8 dans laquelle l'organe de raidissement comporte un fil solide présentant une section transversale circulaire.

21. Sonde à ballonnet selon la revendication 8 dans laquelle l'organe de raidissement comporte une lumière conçue pour qu'un guide-fil puisse y passer.

22. Sonde à ballonnet selon la revendication 8 dans laquelle l'organe de raidissement a une première propriété physique à un premier emplacement et une deuxième propriété physique à un deuxième emplacement, la première propriété physique étant différente de la deuxième propriété physique.

23. Sonde à ballonnet selon la revendication 22 dans laquelle la première propriété physique comporte une première rigidité et la deuxième propriété physique comporte une deuxième rigidité, la première rigidité étant supérieure à la deuxième rigidité, et le premier emplacement étant proximal par rapport au deuxième emplacement.

24. Sonde à ballonnet selon la revendication 22 dans laquelle l'organe de raidissement présente une section transversale conique.
